# EUROPEAN PATENT APPLICATION

(11) **EP 4 799 625 A1**
(43) Date of publication of application: **02.09.2026**
(21) Application number: 25160662.0
(22) Date of filing: 27.02.2025
(51) Int. Cl.: A61K 31/4985, A61P 25/00, A61P 25/22, A61P 25/24

(54) **FEZOLINETANT FOR USE IN THE TREATMENT AND PREVENTION OF ANXIETY, FEAR-BASED DISORDERS AND DEPRESSIVE DISORDERS**

(71) Applicant: Universitat Autónoma de Barcelona, 08193 Bellaterra (Cerdanyola del Vallès) Barcelona (ES); Institució Catalana de Recerca I Estudis Avançats, 8010 Barcelona (ES)
(72) Inventor: ANDERO GALI, RAUL, 08193 Bellaterra (Cerdanyola del Vallès) (ES); GALLIO FRONZA, MARIANA, 08193 Bellaterra (Cerdanyola del Vallès) (ES)

(57) **Abstract**

The invention relates to the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone (Fezolinetant) for use as memory modulator in the treatment and/or prevention of anxiety disorders, fear-based disorders and depressive disorders.

## Description

The invention relates to the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone (Fezolinetant) for use as memory modulator in the treatment and/or prevention of anxiety disorders, fear-based disorders and depressive disorders.

### BACKGROUND ART

Fear-based disorders such as specific phobias, panic disorder, and posttraumatic stress disorder (PTSD) are highly prevalent affecting (Bandelow, B., Michaelis, S., 2015. Epidemiology of anxiety disorders in the 21st century. Dialogues Clin. Neurosci. 17, 327-335; Koenen et al., 2017. Posttraumatic stress disorder in the World Mental Health Surveys. Psychol. Med. 47, 2260-2274) around a third of the population during their lifetime. Current treatments for fear-based disorders involve psychotherapy, antidepressants, and anxiolytics (Velasco et al., 2019. Sex differences in fear extinction. Neurosci. Biobehav. Rev. 103, 81-108.). However, their success is limited because they are not effective for all individuals, and new therapies and prevention strategies are necessary to decrease the prevalence of fear-based disorders.

The tachykinin 2 (Tac2) gene encode the neuropeptide neurokinin B (NkB) which preferentially binds to the Neurokinin 3 receptor (NK3R) modulating fear memory) (Bonner et al., 1987. A cDNA encoding the precursor of the rat neuropeptide, neurokinin B. Brain Res. 388, 243-249; Khawaja, A.M., Rogers, D.F., 1996. Tachykinins: receptor to effector. Int. J. Biochem. Cell Biol. 28, 721-738)). The Nk3R antagonist Osanetant has been shown to modulate fear memory in both male and female mice (Florido et al. 2021. Sex differences in fear memory consolidation via Tac2 signaling in mice. Nat. Commun. 12, 2496) suggesting it could be potentially beneficial for the treatment of fear-based disorders. Additionally, the Tac2 pathway has been shown to modulate fear memory in healthy men and women (Florido et al., 2024. Sex differences in neural projections of fear memory processing in mice and humans. Sci. Adv. 10, eadk3365). Nevertheless, Osanetant is not currently approved as a treatment for human diseases.

Fezolinetant corresponds to (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone and is described in WO2014154895.

Fezolinetant was approved by Astellas Pharma for medical use as a selective antagonist of NK-3 receptor and is useful as a therapeutic compound in the treatment and/or prevention of sex-hormone-dependent diseases, including hot flashes in menopausal women.

Fezolinetant besides having affinity with Nk3R, is that it also possesses low affinity with NK1 receptors. For instance, PTSD patients exhibit elevated cerebrospinal fluid levels of substance P, the preferred ligand of Nk1R (Frick, A., Ahs, F., Linnman, C., Jonasson, M., Appel, L., Lubberink, M., Långström, B., Fredrikson, M., Furmark, T., 2015. Increased neurokinin-1 receptor availability in the amygdala in social anxiety disorder: a positron emission tomography study with [11C]GR205171. Transl. Psychiatry 5, e597), and amygdala Nk1R availability is linked to anxiety-based personality traits (Hoppe JM, Frick A, Åhs F, Linnman C, Appel L, Jonasson M, Lubberink M, Långström B, Frans Ö, von Knorring L, Fredrikson M, Furmark T. Association between amygdala neurokinin-1 receptor availability and anxiety-related personality traits. Transl Psychiatry. 2018 Aug 28;8(1):168).

Fear-based disorders involve negative changes in thinking and mood, often resembling depressive symptoms, and are highly comorbid with major depressive disorders. Nk3R antagonists have been proven to possess antidepressant efficacy in five randomized clinical trials (Rupniak and Kramer et al., 2017. NK1 receptor antagonists for depression: Why a validated concept was abandoned. J. Affect. Disord. 223, 121-125).

Accordingly, it would be desirable to have a medicament which enhances the Nk3R receptor blockade effect in fear memory processing in order to improve the treatment and/or prevention of anxiety disorders, fear-based disorders and depressive disorders.

### SUMMARY OF THE INVENTION

A first aspect of the present invention relates to compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone (Fezolinetant) for use in the treatment and/or prevention of anxiety disorders, fear-based disorders or depressive disorders.

According to the invention, the "anxiety disorders" include, among others, the anxiety disorders disclosed by Ströhle et al. (see Ströhle, A., Gensichen, J., Domschke, K., 2018. The Diagnosis and Treatment of Anxiety Disorders. Dtsch. Arztebl. Int. 155, 611-620) and Park et al. (see Park, S.-C., Kim, Y.-K., 2020. Anxiety Disorders in the DSM-5: Changes, Controversies, and Future Directions. Adv. Exp. Med. Biol. 1191, 187-196), particularly, generalized anxiety disorder, social anxiety disorder and childhood-separation anxiety.

Accordingly, in another embodiment the invention relates to compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use defined above, wherein the anxiety based disorders are selected from generalized anxiety disorder, social anxiety disorder and childhood-separation anxiety.

According to the invention the "fear-based disorders" include, among others, the fear-based disorders disclosed by Kogan et al. (see Kogan et al., 2016. The Classification of Anxiety and Fear-Related Disorders in the ICD-11. Depress Anxiety. 2016 Dec;33(12):1141-1154.) and Milad et al., (see Milad, M.R., et al. 2014. Neuroscience of fear extinction: implications for assessment and treatment of fear-based and anxiety related disorders. Behav Res Ther.62:17-23.), particularly phobias, panic disorder, obsessive-compulsive disorder and posttraumatic stress disorder (PTSD).

In another embodiment the invention relates to compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use defined above, wherein the fear-based disorders are selected from phobias, panic disorder, obsessive compulsive disorder and posttraumatic stress disorder (PTSD).

According to the invention, the "depressive disorders" include, among others, all the depressive disorders described by Angst and Merikangas (see Angst, J., Merikangas, K., 1997. The depressive spectrum: diagnostic classification and course. J. Affect. Disord. 45, 31-40) and Klein et al. (see Klein, D.N., 2008. Classification of depressive disorders in the DSM-V: proposal for a two-dimension system. J. Abnorm. Psychol. 117, 552-560)., particularly, major depressive disorder, bipolar disorder, melancholia, psychotic depression, dysthymic disorder, seasonal depression and antenatal and postnatal depression.

Accordingly, in another embodiment the invention relates to the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use as defined above, wherein the depressive disorders are selected from major depressive disorder, bipolar disorder, melancholia, psychotic depression, dysthymic disorder, seasonal depression and antenatal and postnatal depression.

Another aspect of the invention relates to the use of the compound as memory modulator.

Another aspect of the invention relates to the use of the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the manufacture of a medicament for treatment and/or prevention of anxiety disorders, fear-based disorders or depressive disorders.

Another aspect of the invention relates to a method of treatment and/or prevention of anxiety disorders, fear-based disorders or depressive disorders, especially a human being, which comprises administering to said subject the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone.

The present invention also relates to a pharmaceutical composition that comprises the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone (or a pharmaceutically acceptable salt or solvate thereof) and one or more pharmaceutically acceptable excipients. The excipients must be "acceptable" in the sense of being compatible with the other ingredients of the composition and not deleterious to the recipients thereof.

Thus, another aspect of the invention relates to a pharmaceutical composition which comprises the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use defined above, or a pharmaceutically acceptable salt thereof; and one or more pharmaceutically acceptable excipients.

The compounds of the present invention can be administered in the form of any pharmaceutical formulation, the nature of which, as is well known, will depend upon the nature of the active compound and its route of administration. Any route of administration may be used, for example oral, parenteral, nasal, ocular, rectal and topical administration.

Solid compositions for oral administration include tablets, granulates and capsules. In any case the manufacturing method is based on a simple mixture, dry granulation or wet granulation of the active compound with excipients. These excipients can be, for example, diluents such as lactose, microcrystalline cellulose, mannitol or calcium hydrogen phosphate; binding agents such as for example starch, gelatin or povidone; disintegrants such as sodium carboxymethyl starch or sodium croscarmellose; and lubricating agents such as for example magnesium stearate, stearic acid or talc. Tablets can be additionally coated with suitable excipients by using known techniques with the purpose of delaying their disintegration and absorption in the gastrointestinal tract and thereby provide a sustained action over a longer period, or simply to improve their organoleptic properties or their stability. The active compound can also be incorporated by coating onto inert pellets using natural or synthetic film-coating agents. Soft gelatin capsules are also possible, in which the active compound is mixed with water or an oily medium, for example coconut oil, mineral oil or olive oil.

Powders and granulates for the preparation of oral suspensions by the addition of water can be obtained by mixing the active compound with dispersing or wetting agents; suspending agents and preservatives. Other excipients can also be added, for example sweetening, flavoring and coloring agents.

Liquid forms for oral administration include emulsions, solutions, suspensions, syrups and elixirs containing commonly-used inert diluents, such as purified water, ethanol, sorbitol, glycerol, polyethylene glycols (macrogols) and propylene glycol. Said compositions can also contain coadjuvants such as wetting, suspending, sweetening, flavoring agents, preservatives and buffers.

Injectable preparations, according to the present invention, for parenteral administration, comprise sterile solutions, suspensions or emulsions, in an aqueous or non-aqueous solvent such as propylene glycol, polyethylene glycol or vegetable oils. These compositions can also contain coadjuvants, such as wetting, emulsifying, dispersing agents and preservatives. They may be sterilized by any known method or prepared as sterile solid compositions, which will be dissolved in water or any other sterile injectable medium immediately before use. It is also possible to start with sterile materials and keep them under these conditions throughout the whole manufacturing process.

For the rectal administration, the active compound can be preferably formulated as a suppository on an oily base, such as for example vegetable oils or solid semisynthetic glycerides, or on a hydrophilic base such as polyethylene glycols (macrogol).

The compounds of the invention can also be formulated for their topical application for the treatment of pathologies occurring in zones or organs accessible through this route, such as eyes, skin and the intestinal tract. Formulations include creams, lotions, gels, powders, solutions and patches wherein the compound is dispersed or dissolved in suitable excipients.

For nasal administration or for inhalation, the compound can be formulated as an aerosol, and it can be conveniently released using suitable propellants.

The dosage and frequency of doses will depend upon the nature and severity of the disease to be treated, the age, the general condition, and body weight of the patient, as well as the particular compound administered and the route of administration, among other factors.

Throughout the present specification, the term "treatment" means eliminating, reducing or ameliorating the cause or the effects of a disease. For purposes of this invention treatment includes, but is not limited to, alleviation, amelioration or elimination of one or more symptoms of the disease; diminishment of the extent of the disease; stabilized (i.e. not worsening) state of disease; delay or slowing of disease progression; amelioration or palliation of the disease state; and remission of the disease (whether partial or total).

As used herein, the term "prevention" refers to preventing the occurrence of a disease in a subject that is predisposed to or has risk factors but does not yet display symptoms of the disease. Prevention includes also preventing the recurrence of a disease in a subject that has previously suffered said disease.

According to the invention, the term "memory modulator" refers to an agent that alters the mnemonic process by either impairing or enhancing memory consolidation and retrieval.

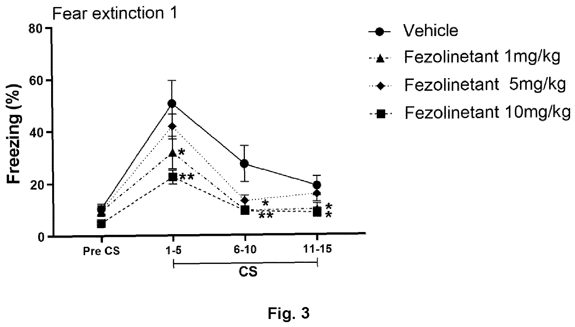

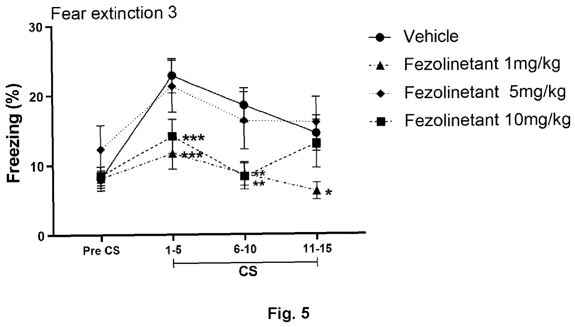

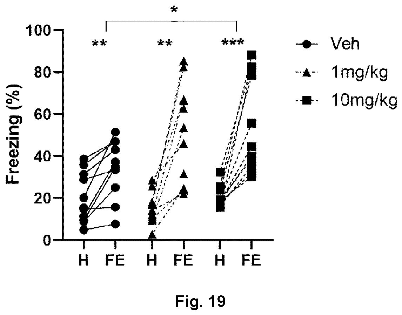

Considering the results shown in the present invention, Fezolinetant (1 and 10mg/kg) treatment impairs fear memory consolidation in males while increasing memory consolidation in females, in the proestrus phase of the estrous cycle. Thus, the invention describes methods for enhancing beneficial memory retention or diminishing maladaptive fear memory to reduce the risk of psychiatric disorders such as depressive, anxiety, and fear-related disorders. The invention proposes the use of Fezolinetant as follows:
1) For Men: Fezolinetant is administered shortly after a traumatic event (e.g., a car accident) to diminish maladaptive fear memories. This intervention aims to reduce the likelihood of developing psychiatric disorders such as PTSD, depression, or other fear-related disturbances. This conclusion is supported by Figure 3 and 5, which demonstrate that the treatment with Fezolinetant at 1 and 10mg/kg can reduce the freezing percentual to the tone when compared to mice treated with vehicle.
2) For Women: Fezolinetant is administered following a successful psychotherapy session to enhance the consolidation of beneficial therapeutic memories. This approach aims to maximize the lasting positive effects of psychotherapy. This effect can be observed in Figure 19, where it's possible to verify that females in proestrus cycle when treated with Fezolinetant (10mg/kg) showed increased percentual of freezing when compared to vehicle group which denotes enhanced memory.
   However, given that these findings are based on preclinical results, we cannot guarantee that the same sex-dependent effects will occur in humans. Considering the hormonal fluctuations and the longer menstrual cycle in women, it is also possible that both sexes may respond similarly to Fezolinetant in humans. This could result in either impaired or enhanced memory following its administration. The Fezolinetant could be administered alone or in combination with antidepressants to achieve superior efficacy through pharmacological synergism. The antidepressant drugs include the classes and respective molecules: 1) selective reuptake serotonin inhibitors: Sertraline, Fluoxetine, Escitalopram, Citalopram, Paroxetine; 2) Serotonin-Norepinephrine Reuptake Inhibitors (SNRIs): Duloxetine, Venlafaxine and Desvenlafaxine; 3) Atypical Antidepressants: Bupropion, Mirtazapine and Trazodone; 4) Tricyclics: Amitriptyline, Nortriptyline, Imipramine and Clomipramine; 5) Monoamine oxidase inhibitors: Phenelzine, Selegiline and Tranylcypromine; 6) Others: Esketamine, Brexanolone, and Gepirone.

The Fezolinetant could be administered alone or in combination with anxiolytic drugs to achieve superior efficacy through pharmacological synergism. The anxiolytic drugs include the classes and respective molecules: 1) Benzodiazepines: Alprazolam, Diazepam, Lorazepam, Clonazepam, Chlordiazepoxide; 2) Azapirones: Buspirone; 3) Barbiturates: Phenobarbital, Amobarbital; 4) Beta-Blockers: Propranolol; 5) Atypical Antipsychotics: Risperidone, Quetiapine; 6) Anticonvulsants: Gabapentin and Pregabalin.

Accordingly, another aspect of the invention relates to the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use defined above or to a pharmaceutically acceptable salt thereof, which is administered alone or in combination with one or more antidepressant or one or more anxiolytic drugs.

The present invention demonstrated that Fezolinetant treatment has a non-monotonic effect, with a typical U-shaped dose-response curve, presenting effectivity at the lowest and highest doses (1 and 10mg/kg) but not at the intermediate dose (5mg/kg) in both males and females. In contrast, Osanetant in males and females modulated fear memory consolidation at a dose of 5mg/kg, presenting no effects at 1 and 10mg/kg, and therefore possessing an inverted U-shaped dose-response curve, suggesting different mechanisms of action.

The present invention demonstrates that Fezolinetant effectively attenuates the recall of fear memories and facilitates extinction at a dosage of 1 mg/kg (Figures 3 and 5), suggesting greater efficacy since is five times lower than previous works which showed that Osanetant can attenuate fear memory recall at 5 mg/kg (Andero, R., Dias, B.G., Ressler, K.J., 2014; A role for Tac2, NkB, and Nk3 receptor in normal and dysregulated fear memory consolidation. Neuron. 83(2):444-454. Florido, A., Perez-Caballero, L., Velasco, E.R., Molina, P., Marin-Blasco, I., Andero, R., 2021. Direct and Indirect Measurements of Sex Hormones in Rodents During Fear Conditioning. Curr. Protoc. 1, e102).

Further, Fezolinetant was significantly effective in attenuating the fear memory recall related to trauma during three sessions of fear memory extinction, which indicates a long-lasting effect by Osanetant treatment.

Furthermore, fear-related disorders involve negative changes in thinking and mood, often resembling depressive symptoms, and are highly comorbid with major depressive disorder, making Fezolinetant useful for its treatment and/prevention over Osanetant.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

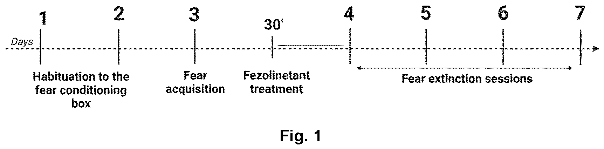

**Fig. 1****.** Experimental protocol used in this investigation. The animals were habituated to the fear conditioning chamber for 5 minutes per day over two consecutive days. On the third day of the protocol, the animals underwent a fear acquisition session, which consisted of 5 tones (30 s duration, 6 kHz, 75 dB) that coterminated with a footshock (1 s, 0.3 mA). Thirty minutes after the session, they were administered either a vehicle (5% Tween 80 and 5% DMSO in saline) or Fezolinetant (1, 5, or 10 mg/kg, intraperitoneally, (i.p.)). Twenty-four hours later, the mice were assessed in the fear extinction (FE) session, during which 15 tones were presented without the electric shock. This extinction protocol was repeated for the next three days (FE1-4).

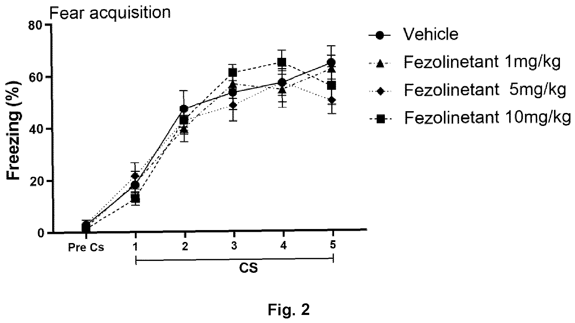

**Fig. 2****.** Freezing percentual of male mice in response to the delivery of tones paired with electric shocks during fear acquisition. Data are presented as mean ± SEM for 11-12 independent animals.
**Fig. 3****.** Effect of Fezolinetant (1, 5, and 10 mg/kg, i.p.) administered 30 minutes after fear acquisition in male mice subjected to the first fear extinction (FE1) session. Data are presented as mean ± SEM for 11-12 independent animals. ** p < 0.01, and *** p < 0.001 compared to the vehicle group (two-way repeated measures ANOVA followed by the LSD test).

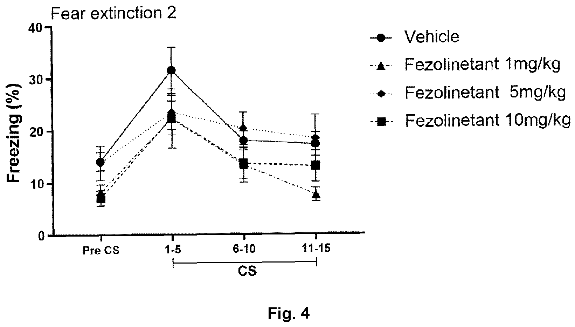

**Fig. 4****.** Effect of Fezolinetant (1, 5, and 10 mg/kg, i.p.) administered 30 minutes after fear acquisition in male mice subjected to the second fear extinction (FE2) session. Data are presented as mean ± SEM for 11-12 independent animals.
**Fig. 5****.** Effect of Fezolinetant (1, 5, and 10 mg/kg, i.p.) administered 30 minutes after fear acquisition in male mice subjected to the third fear extinction (FE3) session. Data are presented as mean ± SEM for 11-12 independent animals. * p < 0.05, ** p < 0.01, and *** p < 0.001 compared to the vehicle group (two-way repeated measures ANOVA followed by the LSD test).

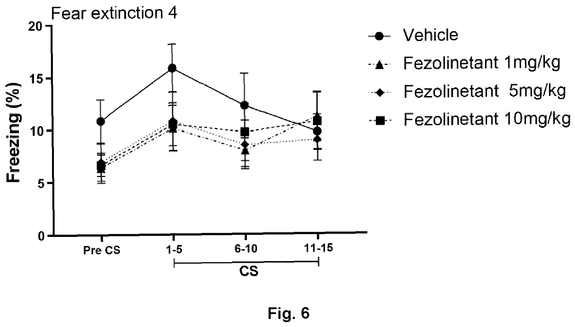

**Fig. 6****.** Effect of Fezolinetant (1, 5, and 10 mg/kg, i.p.) administered 30 minutes after fear acquisition in male mice subjected to the fourth fear extinction (FE4) session. Data are presented as mean ± SEM for 11-12 independent animals. ** p < 0.01, and *** p < 0.001 compared to the vehicle group (two-way repeated measures ANOVA followed by the LSD test).

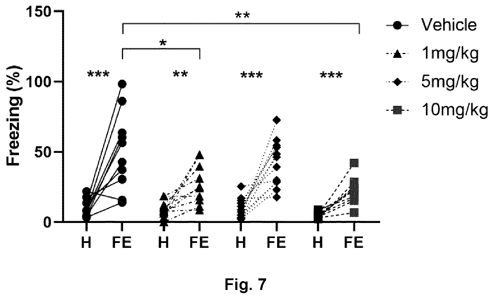

**Fig. 7****.** Fear expression of male mice during the first 5 Cs of FE1. Data are presented as mean ± SEM for 11-12 independent animals. *** p < 0.01 and* *** p < 0.001 when compared to the habituation session (paired T-test ) or ** p < 0.05 and **** p < 0.01 when compared to the vehicle group (unpaired T-test).

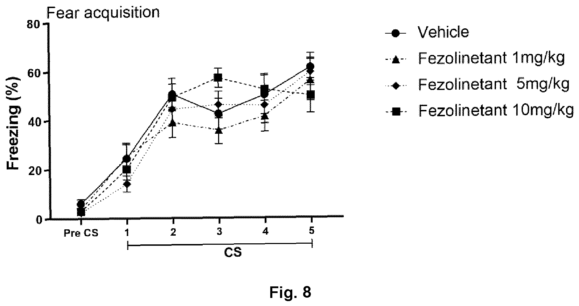

**Fig. 8****.** Freezing percentual of female mice in response to the delivery of tones paired with electric shocks during fear acquisition. Data are presented as mean ± SEM for 11-12 independent animals.

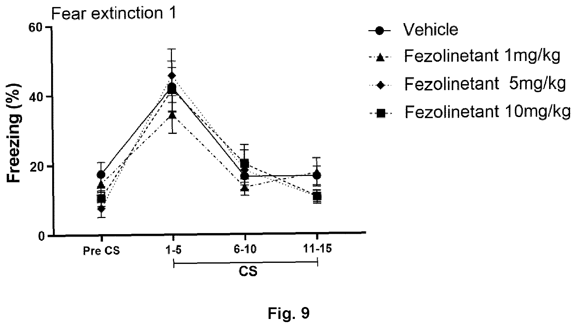

**Fig. 9****.** Effect of Fezolinetant (1, 5, and 10 mg/kg, i.p.) administered 30 minutes after fear acquisition in female mice subjected to the first fear extinction (FE1) session. Data are presented as mean ± SEM for 11-12 independent animals.

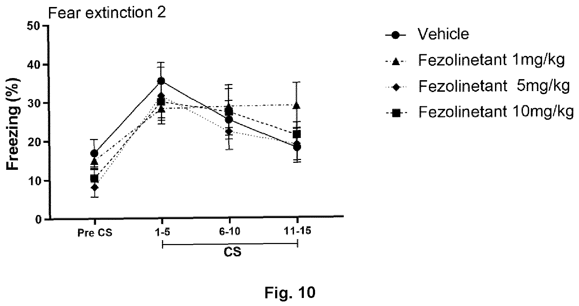

**Fig. 10****.** Effect of Fezolinetant (1, 5, and 10 mg/kg, i.p.) administered 30 minutes after fear acquisition in female mice subjected to the second fear extinction (FE2) session. Data are presented as mean ± SEM for 11-12 independent animals.

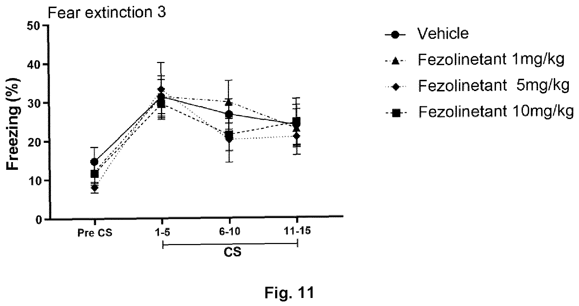

**Fig. 11****.** Effect of Fezolinetant (1, 5, and 10 mg/kg, i.p.) administered 30 minutes after fear acquisition in female mice subjected to the third fear extinction (FE3) session. Data are presented as mean ± SEM for 11-12 independent animals.

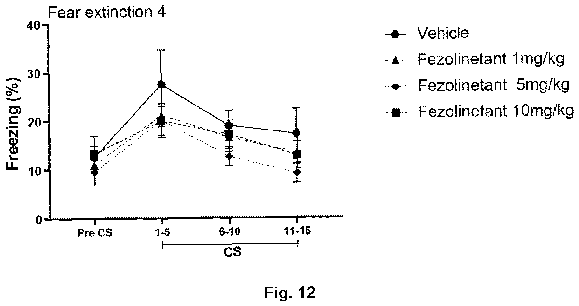

**Fig. 12****.** Effect of Fezolinetant (1, 5, and 10 mg/kg, i.p.) administered 30 minutes after fear acquisition in female mice subjected to the fourth fear extinction (FE4) session. Data are presented as mean ± SEM for 11-12 independent animals.

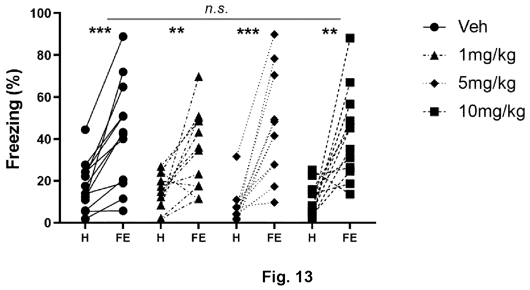

**Fig. 13****.** Fear expression of female mice during the first 5 Cs of FE1. Data are presented as mean ± SEM for 11-12 independent animals. *** p < 0.01 and* *** p < 0.001 when compared to the habituation session (paired T-test) or ** p < 0.05 and* *** p < 0.01 when compared to the vehicle group (unpaired T-test).

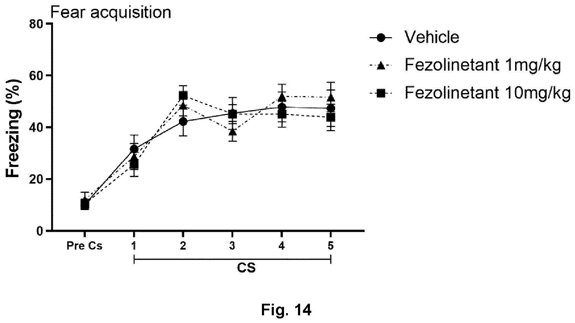

**Fig. 14****.** Freezing percentual of female mice, exclusively in the proestrus phase of the estrous cycle, in response to the delivery of tones paired with electric shocks during fear acquisition. Data are presented as mean ± SEM for 11-12 independent animals.

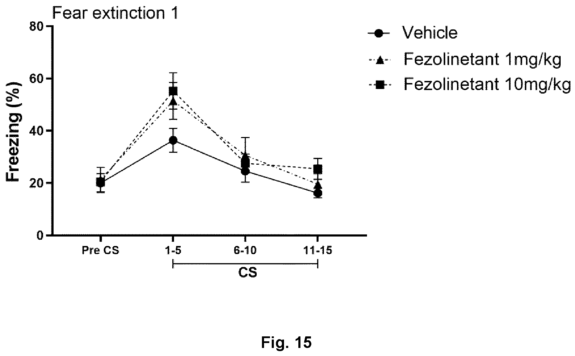

**Fig. 15****.** Effect of Fezolinetant (1 and 10 mg/kg, i.p.) administered 30 minutes after fear acquisition in female mice, exclusively in the proestrus phase of the estrous cycle, subjected to the first fear extinction (FE1) session. Data are presented as mean ± SEM for 11-12 independent animals.

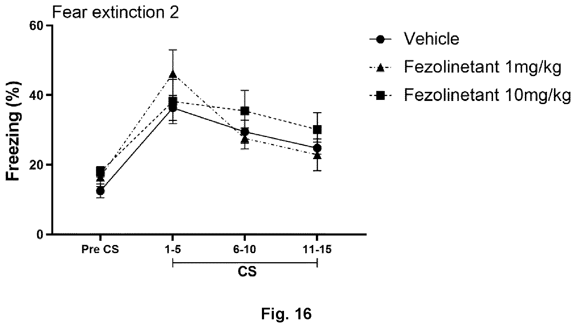

**Fig. 16****.** Effect of Fezolinetant (1 and 10 mg/kg, i.p.) administered 30 minutes after fear acquisition in female mice, exclusively in the proestrus phase of the estrous cycle, subjected to the second fear extinction (FE2) session. Data are presented as mean ± SEM for 11-12 independent animals.

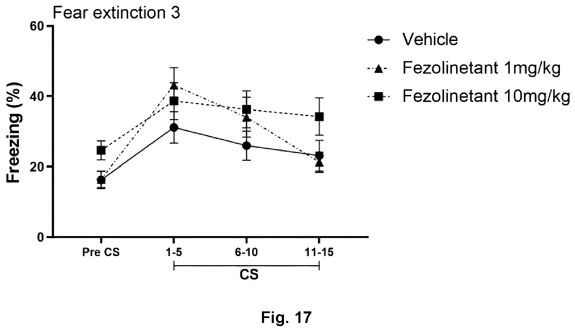

**Fig. 17****.** Effect of Fezolinetant (1 and 10 mg/kg, i.p.) administered 30 minutes after fear acquisition in female mice, exclusively in the proestrus phase of the estrous cycle, subjected to the third fear extinction (FE3) session. Data are presented as mean ± SEM for 11-12 independent animals.

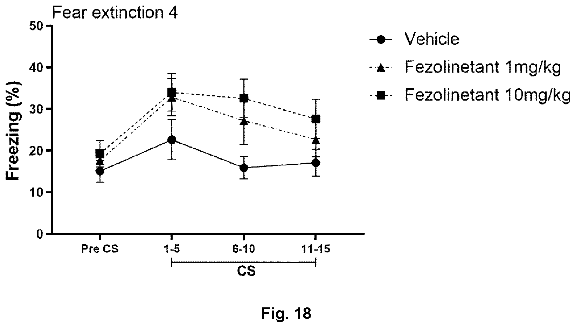

**Fig. 18****.** Effect of Fezolinetant (1 and 10 mg/kg, i.p.) administered 30 minutes after fear acquisition in female mice, exclusively in the proestrus phase of the estrous cycle subjected to the fourth fear extinction (FE4) session. Data are presented as mean ± SEM for 11-12 independent animals.
**Fig. 19****.** Fear expression of female mice, exclusively in the proestrus phase of the estrous cycle, during the first 5 Cs of FE1. Data are presented as mean ± SEM for 11-12 independent animals. ** *p < 0.01 and* *** p < 0.001 when compared to the habituation session (paired T-test ) or ** p < 0.05 and **** p < 0.01 when compared to the vehicle group (unpaired T-test).

### Examples

### Materials and methods

### 1. Drugs

Fezolinetant (Cat. HY-19632, MedChemExpress, USA) was administered intraperitoneally (i.p.) at the doses 1, 5, and 10mg/kg using 5% Tween 80 5% DMSO in saline as the vehicle, 30 minutes after the fear acquisition (FA).

### 2. Mice

All experiments were conducted using adult male (8 weeks old) C57BL/6J mice (Charles River Laboratories). The animals were housed in groups of four under standard conditions, including a 12:12 h light/dark cycle (lights on from 8:00 am to 8:00 pm), with a controlled temperature of 22 ± 1 °C and humidity (~40%). Behavioral procedures and pharmacological manipulations began at 8:00 am. All procedures were approved by the Ethics Committee of the Universitat Autònoma de Barcelona and the Generalitat de Catalunya (Ref: CEEAH 3603) and were carried out in accordance with the European Communities Council Directive (2010-63-UE) and Spanish legislation (RD 53/2013).

### 3. Cued-Fear Conditioning

All stages of Cued-Fear Conditioning test - Habituation, FA, and Fear Extinction (FE) were performed in a fear chamber consisting of a black methacrylate box with a transparent front door (25 × 25 × 25 cm³) inside a sound-attenuating chamber (67 × 53 × 55 cm³) with a computerized Startle system (Panlab-Harvard, Barcelona, Spain)· Delivery of tones and shocks was simultaneously controlled by Packwin v2.0 software (Panlab-Harvard, Barcelona, Spain).

Animals were habituated to the chambers for 5 min/day for two consecutive days before FA. For cue-dependent fear conditioning, all animals remained 5 min in the fear chamber before the onset of the first tone. During FA, all groups received 5 trials consisting of a tone as the Conditioned Stimulus (CS) (30 s, 6 kHz, 75 dB) that coterminated with a footshock which served as the Unconditioned Stimulus (US) (1 s, 0.3 mA). The intertrial interval (ITI) was 3 min, and 3 additional min followed the last trial. All animals received treatments 30 min after FA to manipulate the consolidation of memory and therefore avoid any effect during acquisition. The first FE test was performed 24 h after FA, and repeated for other 2 consecutive days. Mice remained 5 min in the chamber before trials and afterward were exposed to 15 trials of the 30 s CS tone alone (cued-fear) with a 0.5 min of ITI interval. An additional 0.5 min interval followed the last trial of FE. Freezing behavior, defined as the complete absence of movement except for respiration, was analyzed using ezTrack (Pennington et al., 2021). To ensure that freezing behavior was exclusive of the previous tone-shock conditioning, different contexts were utilized for FA and FE. FA context consisted of a yellow light source (~10 lux), a grid floor of 25 bars (3 mm Ø and 10 mm between bars) that dispensed the footshocks, background noise of 60 dB produced by a ventilation fan and a solution of ethanol (EtOH) 70% (v/v) odor was used for cleaning between sessions. FE context consisted of a red light source (~10 lux), a gray floor covering the bars, no background noise, and CR36-bronopol 0.26 % (v/v), benzalkonium chloride 0.08% (v/v), and isopropyl alcohol 41% (v/v) (José Collado, Barcelona, Spain) for cleaning, with changes in the length and turns of the transportation route from the vivarium to the testing room between FA and FE.

### 4. Vaginal smears cytology

Determination of the estrous stage in females was performed by assessing vaginal smear cytology as described by Florido et al. (2021a). Before the fear acquisition, female mice had the estrous cycle assessed for 2 consecutive cycles (approximately 8-10 days) for habituation. The vaginal lavage was performed with a 20 µl pipette loaded with 10 µl of standard NaCl 0.9% (w/v) solution, and later the tip was softly placed on the vaginal aperture. In case of urination when grabbing the animal, urine was cleaned using regular tissue paper. The 10 µl of saline was unloaded and collected 5 consecutive times and placed on an adhesion slide (Superfrost Plus, Avantor-VWR, Pensilvanya, EUA). All vaginal smear samples were collected between 8:00 and 10:00 am. Slides were dried using a hot plate (RT2 Advanced, Thermo Fisher Scientific, Massachusetts, EUA) at 37 °C for 30 min and later stained in Cresyl Violet Acetate (C5042, Sigma-Aldrich, Spain) 0.1% (v/v), washed twice for 1 min in distilled water and read in brightfield microscopy with a 10× or 20× objective in an Eclipse Ei microscope (Zeiss, Spain). The different stages of the estrous cycle were assessed depending on the proportion of cornified epithelial cells, round nucleated epithelial cells, or leukocytes. The female mice were submitted to Fear Acquisition when in Proestrus phase, which is characterized by a high proportion (>80%) of nucleated epithelial cells, which might present small amounts of cornified epithelial cells. Conditioned animals were housed separately from non-conditioned animals.

### 5. Statistical analysis

Statistical analyses were performed using IBM SPSS Statistics 27.0, and graphs were generated using GraphPad 9.0. Trials from FA, or the mean of groups of 4 trials for FE tests, were analyzed using two-way repeated measures ANOVA. The Least Significant Difference (LSD) post-hoc test was applied to evaluate overall treatment effects and individual differences in cases of significant factor interactions. The results of fear expression were analysed by Paired T-Test when compared the freezing time in habituation x the first five CS of each group. Unpaired T-Test was used when comparing the fear expression between treatment groups. Results are presented as means ± SEM for 11-12 animals per group, assessed in two replicates of 5-6 animals per group. Statistical significance was set at P ≤ 0.05. Outlier detection was conducted using Grubb's test, and outliers were removed when appropriate.

### Example 1

In males, as shown in Figure 2, all animals subjected to the fear acquisition (FA) session of the cued-fear conditioning paradigm demonstrated an equal ability to acquire fear memory, as evidenced by the increasing freezing rate during the presentation of the tones paired with the electric shocks (two-way repeated measures ANOVA for time x treatment: F(12,172)=1.051, p=0.405). After 24 hours, during the fear extinction (FE) session 1, in which the tones are presented in the absence of the electric shock, the systemic administration of Fezolinetant at doses of 1 and 10 mg/kg resulted in a lower freezing rate compared to the vehicle group from Cs1-15 (Figure 3) (time x treatment: F(6,80)=2,403, p=0.035). A significant effect of the 5 mg/kg dose was detected only at Cs 7-9, suggesting that the 1 and 10 mg/kg doses promote a more robust attenuation of fear expression.

In FE2, no significant interaction between time and treatment was observed (main effect of time: F(2,84)=17.309, p<0.001; main effect of treatment: F(3,42)=2.563, p=0.048) and therefore the results of the posthoc test are not shown (Figure 4). During FE3, the groups treated with Fezolinetant at doses of 1 and 10 mg/kg showed a lower freezing rate when compared to the vehicle group (time x treatment: F(6,82)=2.602, p=0.003) (Figure 5). This suggests that Fezolinetant treatment enhances the retention of extinction memory. However, no statistically significant effects were observed with the 5 mg/kg dose of Fezolinetant. As shown in Figure 6, in FE4 no differences were observed among the groups (time x treatment: F(6,80)=1.447, p=0.207). When investigating the fear expression as the freezing time in the first 5 tones of FE1 when compared to the habituation time preceding the Cs, all the groups successfully learned the fear (paired T-test: [Vehicle: t=4.258, df=9, p=0.0021], [1mg/kg: t=3.653, df=9, p=0.0053], [5mg/kg: t=5.630, df=11, p= 0,0002] and [10mg/kg: t=6.457, df=10, p<0.001] (Figure 7). When comparing the fear expression between groups the animals treated with 1 and 10 mg/kg showed statistically significant lower freezing time when compared to the vehicle group (unpaired T-test: [1mg/kg: t=2.353, df=18, p=0.03], [5mg/kg: t=0.9149, df=20, p=0.3711] and [10mg/kg: t=3.175, df=19, p= 0.005].

When the effect of Fezolinetant treatment was assessed in female mice, no differences were found during FA (time x treatment: F(12,176)=1.443, p=0.171) (Figure 8). In the opposite direction of the results obtained with male mice, the treatment with Fezolinetant in the same dosage did not reduce the freezing response to the tones in FE1-4 (time x treatment: FE1 (F(6,82)=1.508, p=0.203), FE2 (F(6,86)=1.424, p=0.215), FE3 (F(6,86)=1.024, p=0.414) and FE4 (F(6,86)= 0.278, p=0.946) (Figure 9, 10, 11 and 12). When assessing the fear expression, all groups learned the fear equally (paired T-test: [Vehicle: t=4.876, df=11, p<0.001], [1mg/kg: t=3.451, df=11, p=0.0054], [5mg/kg: t=5.495, df=10, p< 0.001] and [10mg/kg: t=4.015, df=11, p=0.0020]. However, no differences were found between groups in total time freezing during the fear expression (unpaired T-test: [1mg/kg: t=0.817, df=22, p=0.39], [5mg/kg: t=0.2959, df=21, p=0.7702] and [10mg/kg t=0.08563, df=22, p=0.9325] (Figure 13).

Mounting evidence has shown that the estrous cycle modulates the consolidation and extinction of fear memories (see: Milad, M.R., Igoe, S.A., Lebron-Milad, K., Novales, J.E., 2009. Estrous cycle phase and gonadal hormones influence conditioned fear extinction. Neuroscience 164, 887-895; and Milad, M.R., Rosenbaum, B.L., Simon, N.M., 2014. Neuroscience of fear extinction: implications for assessment and treatment of fear-based and anxiety related disorders. Behav. Res. Ther. 62, 17-23). Therefore, it was next investigated if the absence of effect of Fezolinetant treatment in females could be partially attributed to a memory enhancer action in proestrus. Thus, an independent group of mice was first habituated to the vaginal lavage through the assessment of approximately two consecutive cycles. When in the proestrus phase, mice were submitted to fear acquisition and 30 minutes later administered with the Fezolinetant at 1 and 10mg, the effective doses in male mice. As demonstrated in Figure 14 no differences were found in FA (time x treatment: F(8,124)=0.916, p=0.506). In the FE1 (Figure 15) there was a trend to both 1 and 10mg/kg dosages to increase the percentual of the time spent in freezing, however, no statistically significant interaction was found (time x treatment: F(4,64)=2.039, p=0.099). Similarly in the other sessions FE2-4 no differences were found (time x treatment: FE2 (F(4,64)=1.967 p=0.110), FE3 (F(4,62)=1.436, p=0.233) and FE4 (F(4,64)= 0.432, p=0.785) (Figure 16, 17 and 18). As depicted in Figure 19, all groups showed the ability to learn the fear (paired T-test: [Vehicle: t=4.126, df=9, p=0.0026], [1mg/kg: t=4.896, df=8, p=0.0012], and [10mg/kg: t=5,707, df=10, p=0.0002]. Besides not showing significant interaction of time x treatment when analyzing FE1 session by two-way ANOVA of repeated measures, when comparing the fear expression to vehicle group it's possible to verify that females in proestrus cycle when treated with Fezolinetant showed increased percentual of freezing (unpaired T-test: [1mg/kg: t=2.006, df=19, p=0.06], and [10mg/kg t=3.007, df=19, p=0.0073].

## Claims

1. The compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for use in the treatment and/or prevention of fear-based disorders or depressive disorders.

2. The compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use according to claim 1, wherein the fear-based disorders are selected from specific phobias, panic disorder, obsessive-compulsive disorder and posttraumatic stress disorder (PTSD).

3. The compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use according to claim 1, wherein the depressive disorders are selected from major depressive disorder, bipolar disorder, melancholia, psychotic depression, dysthymic disorder, seasonal depression and antenatal and postnatal depression.

4. The compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use according to claim 1, wherein the anxiety disorders are selected from generalized anxiety disorder, social anxiety disorder and childhood-separation anxiety.

5. A pharmaceutical composition which comprises the compound (R)-(4-fluorophenyl)-(8-methyl-3-(3-methyl-1,2,4-thiadiazol-5-yl)-5,6-dihydro-[1,2,4]trizolo[4,3-a]pyrazin-7(8H)-yl)methanone for the use according to any of claims 1 to 3, or a pharmaceutically acceptable salt thereof; and one or more pharmaceutically acceptable excipients.
